# EUROPÄISCHE PATENTANMELDUNG

(11) **EP 3 254 711 A1**
(43) Veröffentlichungstag der Anmeldung: **13.12.2017**
(21) Anmeldenummer: 17173200.1
(22) Anmeldetag: 29.05.2017
(51) Int. Cl.: A61M 1/00

(54) **VAKUUMTHERAPIEGERÄT**

(30) Priorität: 31.05.2016 DE 102016109975
(71) Anmelder: ATMOS MedizinTechnik GmbH & Co. KG, 79853 Lenzkirch (DE)
(72) Erfinder: Greiser, Maik, 79761 Waldshut-Tiengen (DE)
(74) Vertreter: Westphal, Mussgnug & Partner Patentanwälte mbB

(57) **Zusammenfassung**

Bereitgestellt wird ein Vakuumtherapiegerät (1) mit einer Stromversorgung (10), einer Steuerelektronik (60), einer Unterdruckpumpe (70), und einem Anschluss für eine Drainageleitung, an dem mittels der Unterdruckpumpe (70) ein Unterdruck erzeugt werden kann, wobei das Vakuumtherapiegerät (1) eine Kamera (30) aufweist, die in Signalkommunikation mit der Steuerelektronik (60) steht.

## Beschreibung

Die Erfindung betrifft ein Vakuumtherapiegerät mit den Merkmalen des Oberbegriffs des Patentanspruchs 1.

Die Vakuum- oder Unterdrucktherapie, die auch unter dem Akronym NPWT (negative pressure wound therapy) bekannt ist, ist ein weit verbreitetes Verfahren zur Verbesserung der Wundheilung, bei dem eine Wunde mit einem Wundverschluss, z.B. einem Vakuumverband, verschlossen und über ein Drainagesystem mit einer Pumpe zur Erzeugung von Unterdruck in der Wunde verbunden wird. Die Pumpe wird in der Regel über ein Steuergerät angesteuert, so dass ein kontinuierlicher, intermittierender oder konstanter Unterdruck auf die Wunde wirkt, was eine Verbesserung der Wundheilung mit sich bringen kann. Dabei wird aus dem mit dem Wundverschluss abgedeckten Bereich der Wunde Wundsekret über das Drainagesystem abgesaugt und typischerweise in einen Sekrektbehälter, der oft als Einwegartikel ausgeführt wird, aufgenommen.

Zur Durchführung dieser Therapie, die sich üblicherweise über mehrere Tage hinzieht, werden Vakuumtherapiegeräte, die teilweise auch als NPWT-Geräte oder Unterdrucktherapiegeräte bezeichnet werden, wie sie beispielsweise in der WO2011/018132 beschrieben sind, verwendet und bleiben während dieser Zeit in der Regel beim Patienten, der es auch dann, wenn er sich außerhalb des Krankenbettes bewegt, in den meisten Fällen mitführt.

Begleitend zur eigentlichen Therapie müssen deren Verlauf und deren Ergebnisse dokumentiert und in der Krankenakte hinterlegt werden. Gleichzeitig muss auch der Materialverbrauch bei der Durchführung der Vakuumtherapiebehandlung, etwa bei einem Verbandswechsel oder wenn der Sekrektbehälter ausgewechselt werden muss, nachgehalten werden, um am Ende der Behandlung die Behandlungskosten ordnungsgemäß abrechnen zu können. Derzeit erfolgt dies, indem Ärzte und/oder Pflegepersonal von Zeit zu Zeit, typischerweise bei Verbandswechseln, die Wunde fotografieren und ihren Zustand sowie Materialverbrauch aufschreiben, um diese Informationen dann später in der Patientenakte -die heutzutage in der Regel elektronisch in einem Krankenhausinformationssystem geführt wird- zu hinterlegen.

Im hektischen Krankenhausalltag zeigt sich dabei eine erhebliche Fehleranfälligkeit dieses Systems. Wenn beispielsweise ein Pfleger oder eine Ärztin sich plötzlich um einen dringenden Notfall kümmern müssen, kann nach einem solchen Zwischenfall in der Erinnerung die Zuordnung eines Fotos zum richtigen Patienten nicht mehr möglich sein, oder es kommt dazu, dass vergessen wird, dass noch aussteht, das beispielsweise für einen neuen Verband verwendete Verbrauchsmaterial, in der Krankenakte zu notieren, oder es kommt zu Verwechslungen der jeweiligen Krankenakten.

Andererseits kann es auch vorkommen, dass zum Zeitpunkt des Verbandswechsels der Pfleger oder die Ärztin die Kamera, die zur Dokumentation des Heilungsverlaufs benötigt wird, gerade nicht mitführen, etwa weil sie beim letzten Patienten vergessen wurde.

Die Aufgabe der Erfindung besteht daher darin, ein Vakuumtherapiegerät bereitzustellen, das eine zuverlässigere Dokumentation der Behandlung und des Behandlungsaufwands ermöglicht. Diese Aufgabe wird gelöst durch ein Vakuumtherapiegerät mit den Merkmalen des Patentanspruchs 1. Vorteilhafte Weiterbildungen der Erfindung sind Gegenstand der abhängigen Ansprüche. Das erfindungsgemäße Vakuumtherapiegerät weist in bekannter Weise eine beispielsweise als Akku oder Netzteil ausgestaltbare Stromversorgung, eine Steuerelektronik, eine Unterdruckpumpe (also eine Pumpe, mit der ein Unterdruck erzeugt werden kann) und einen Anschluss für eine Drainageleitung, an dem mittels der Unterdruckpumpe ein Unterdruck erzeugt werden kann, insbesondere indem die Unterdruckpumpe mittels der Steuerelektronik angesteuert wird, auf.

Angemerkt sei, dass bei modernen Steuerelektroniken zwar nicht zwingend, aber doch in der Regel auch mindestens ein Prozessor und mindestens ein Speicherbaustein vorgesehen sind, die es erlauben, Steuerprogramme auszuführen, beispielsweise für die Durchführung von vorgegebenen Saugsequenzen oder zur Überwachung von Gerätefunktionen.

Der Anschluss für die Drainageleitung ist dabei zweckmäßigerweise so angeordnet, dass er das Absaugen von Flüssigkeit aus der Drainageleitung in einen Sekretbehälter des Vakuumtherapiegeräts, der auch als ein in eine Sektretbehälteraufnahme des Vakuumtherapiegeräts aufnehmbarer Einweg-Sekretbehälter ausgeführt sein kann, ermöglicht.

Erfindungswesentlich ist, dass das Vakuumtherapiegerät eine vorzugsweise integrierte, bevorzugt als Kameramodul ausgeführte Kamera aufweist, die in Signalkommunikation mit der Steuerelektronik steht. Dementsprechend hat die Steuerelektronik bevorzugt Zugriff auf mittels des Kameramoduls aufgenommene Bilder und kann diese in einem Speicher, der der Steuerelektronik oder dem Kameramodul zugeordnet sein kann, als Dateien ablegen und verwalten. Verwendbar sind dafür auch übliche Kameramodule, wie sie beispielsweise in aktuellen Smartphones verwendet werden, was die Zusatzkosten für das Vakuumtherapiegerät überschaubar hält.

Die Erfinder haben realisiert, dass dadurch, dass das Vakuumtherapiegerät während der Vakuumtherapie in der Regel fest einem Patienten zugeordnet ist, sichergestellt ist, dass dieses Gerät zu jedem Zeitpunkt, zu dem eine Dokumentation des Heilungsprozesses nötig ist, ohnehin beim Patienten ist und somit ein darin eingebautes Kameramodul für die Dokumentation des Verlaufs der Wundheilung unmittelbar verfügbar ist.

Noch vollständiger kann eine Zuordnung zwischen dem Vakuumtherapiegerät und dem Patienten ausgenutzt werden, wenn das Vakuumtherapiegerät eine Schnittstelle zur Kommunikation mit einem Krankenhausinformationssystem aufweist. In diesem Fall können die mit dem Vakuumtherapiegerät aufgenommenen Daten unmittelbar oder zu fest vorgegebenen Zeitpunkten in das Krankenhausinformationssystem eingespeist werden, wobei eine Zuordnung zwischen Vakuumtherapiegerät und Patient ermöglicht, diese Daten auch unmittelbar der richtigen Krankenakte zuzuordnen. Besonders vorteilhaft ist dabei die Verwendung einer als WLAN-Schnittstelle ausgebildeten Schnittstelle.

Um neben der unmittelbaren fotografischen Dokumentation des Heilungsprozesses in der Krankenakte auch den Materialaufwand für die Behandlung, beispielsweise das verwendete Verbandsmaterial unmittelbar für die Abrechnung erfassen zu können, ist es vorteilhaft, wenn das Vakuumtherapiegerät eingerichtet ist, um eine Software zum Erfassen und/oder Scannen von Barcodes auszuführen, die insbesondere auf die erfindungsgemäß am Vakuumtherapiegerät vorgesehene Kamera als Scanner zurückgreifen kann. Derartige Software wird, in der Regel als App realisiert, bereits bei Smartphones zur Nutzung der Kamera als Scanner eingesetzt und erfordert allenfalls minimale Änderungen zum Einsatz im Vakuumtherapiegerät.

Selbstverständlich weist ein Vakuumtherapiegerät Bedienelemente auf, beispielsweise zum Ein- und Ausschalten und zur Auswahl und Einstellung des Betriebsverhaltens, z.B. des Unterdrucks, oder bestimmter Laufzeiten der Unterdruckpumpe. Eine vorteilhafte Weiterbildung der Erfindung sieht aber vor, dass das Vakuumtherapiegerät ein Eingabemittel zur Eingabe von Text aufweist, das optional auch als ein Bedienelement verwendet werden kann. Dies ermöglicht es, neben der bildlichen Dokumentation des Wundheilungsprozesses auch gleich Kommentare eines Pflegers oder einer Ärztin unmittelbar der Krankenakte des entsprechenden Patienten zuzuordnen.

Besonders vorteilhaft ist es dabei, wenn das Vakuumtherapiegerät ein Display, insbesondere ein Display mit einem Touchscreen, aufweist. Damit wird die Einstellung und Kontrolle gewählter Bedienparameter besonders einfach. In aus anderem Zusammenhang bekannter Art und Weise kann ein solches Display mit Touchscreen zugleich als Eingabemittel zur Eingabe von Text dienen, indem eine Tastatur darauf dargestellt wird, deren Tasten durch Berührung an dieser Stelle des Touchscreens betätigt werden, um den entsprechenden Buchstaben einzugeben.

Die Erfindung wird nun anhand von Figuren, die Ausführungsbeispiele darstellen, näher erläutert. Es zeigen:
- Fig. 1:: Eine Frontansicht eines Ausführungsbeispiels für ein Vakuumtherapiegerät, und
- Fig. 2:: ein schematisches Blockschaltbild mit den wesentlichen Komponenten eines Ausführungsbeispiels für ein Vakuumtherapiegerät.

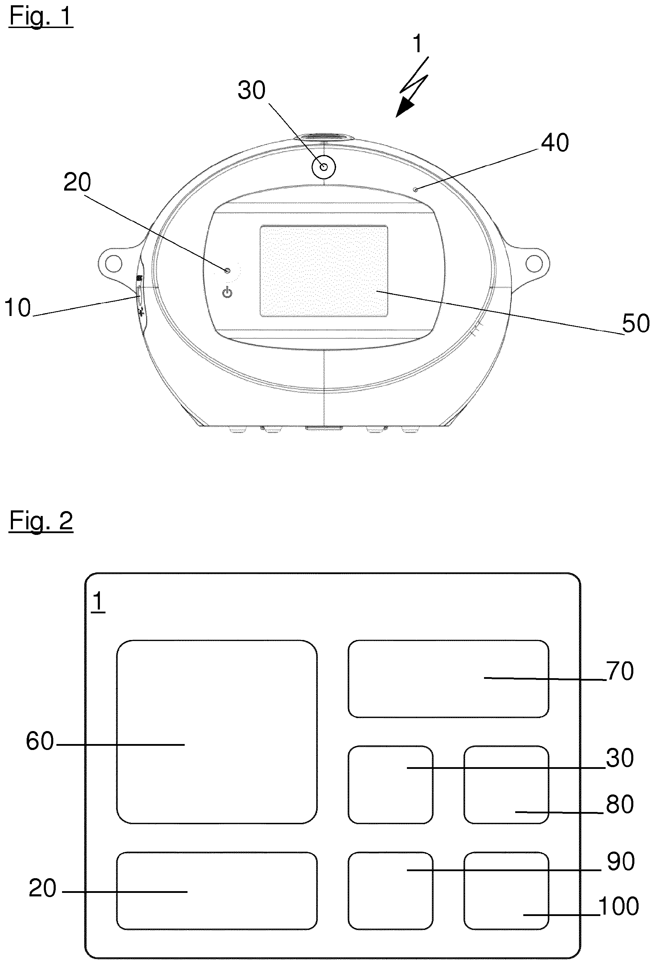

Das in Figur 1 dargestellte Vakuumtherapiegerät 1 weist eine Stromversorgung 10 in Form eines Netzanschlusses, ein Bedienelement 20 in Form eines Ein-/Aus-Schalters, eine integrierte Kamera 30, einen Lichtsensor 40 und ein mit einem Touchscreen ausgerüstetes Display 50 auf. In der dargestellten Ansicht nicht sichtbar ist der Anschluss für eine Drainageleitung.

Der Lichtsensor 40 kann nicht nur eingerichtet sein, um über die Messung der Umgebungshelligkeit z.B. die Beleuchtung des Bildschirm zu beeinflussen, um z.B. zu verhindern, dass der Schlaf des Patienten nachts durch einen hell leuchtenden Bildschirm beeinträchtigt wird, sondern ist in einer bevorzugten Weiterbildung der Erfindung so eingerichtet, dass er der Kamera als Helligkeitssensor dient. Dies kann insbesondere dazu beitragen, bei der fotografischen Dokumentation des Heilungsverlaufs der Wunde die Vergleichbarkeit der einzelnen Fotos zu verbessern.

Wie aus dem Blockschaltbild der Figur 2 hervorgeht, ist das Vakuumtherapiegerät 1 ferner mit einer Steuerelektronik 60, einer Unterdruckpumpe 70 und einer Schnittstelle 80 zu einem Krankenhausinformationssystem, die beispielsweise durch ein WLAN-Modul realisiert sein kann, ausgestattet. Ferner sind optional ein Lautsprecher 90, beispielsweise zur Ausgabe eines akustischen Alarms bei einer Fehlfunktion, und ein USB-Modul 100, mit dem ein Datentransfer von und zu dem Vakuumtherapiegerät 1 bewirkt werden kann, vorhanden.

### Bezugszeichenliste

- 1: Vakuumtherapiegerät
- 10: Stromversorgung
- 20: Bedienelement
- 30: Kamera
- 40: Lichtsensor
- 50: Display
- 60: Steuerelektronik
- 70: Unterdruckpumpe
- 80: Schnittstelle
- 90: Lautsprecher
- 100: USB-Modul

## Patentansprüche

1. Vakuumtherapiegerät (1) mit einer Stromversorgung (10), einer Steuerelektronik (60), einer Unterdruckpumpe (70), und einem Anschluss für eine Drainageleitung, an dem mittels der Unterdruckpumpe (70) ein Unterdruck erzeugt werden kann,
**dadurch gekennzeichnet, dass** das Vakuumtherapiegerät (1) eine Kamera (30) aufweist, die in Signalkommunikation mit der Steuerelektronik (60) steht.

2. Vakuumtherapiegerät (1) nach Anspruch 1,
**dadurch gekennzeichnet, dass** das Vakuumtherapiegerät (1) eine Schnittstelle (80) zur Kommunikation mit einem Krankenhausinformationssystem aufweist.

3. Vakuumtherapiegerät (1) nach Anspruch 2,
**dadurch gekennzeichnet, dass** die Schnittstelle (80) eine WLAN-Schnittstelle ist.

4. Vakuumtherapiergerät (1) nach einem der Ansprüche 1 bis 3, **dadurch gekennzeichnet, dass** das Vakuumtherapiegerät (1) eingerichtet ist, um eine Software zum Erfassen und/oder Scannen von Barcodes auszuführen.

5. Vakuumtherapiegerät (1) nach einem der Ansprüche 1 bis 4, **dadurch gekennzeichnet, dass** das Vakuumtherapiegerät (1) ein Eingabemittel zur Eingabe von Text aufweist.

6. Vakuumtherapiergerät (1) nach einem der Ansprüche 1 bis 5, **dadurch gekennzeichnet, dass** das Vakuumtherapiegerät ein Display (50), insbesondere ein Display (50) mit einem Touchscreen, aufweist.
